# EUROPEAN PATENT APPLICATION

(11) **EP 4 838 541 A1**
(43) Date of publication of application: **11.11.2026**
(21) Application number: 25175552.6
(22) Date of filing: 09.05.2025
(51) Int. Cl.: G16H 50/20, A61B 5/349

(54) **A METHOD FOR ANALYZING A BIOLOGICAL SIGNAL**

(71) Applicant: Technische Universität Dresden Körperschaft des öffentlichen Rechts, 01069 Dresden (DE)
(72) Inventor: Schmidt, Martin, 01277 Dresden (DE); Hammer, Alexander, 01217 Dresden (DE); Malberg, Hagen, 01259 Dresden (DE)
(74) Representative: Riechelmann & Carlsohn Patentanwälte PartG mbB

(57) **Abstract**

The invention relates to a method for analyzing a biological signal, wherein first biological signals measured in a first manner are used to analyze at least one second biological signal measured in a second manner, wherein
- the first biological signals comprise first data, second data and third data, the first data being values measured over a period of time, the second data being values specifying a feature the manner used to measure the first data, and the third data labeling at least one medical or technical state assigned to the first biological signals; and
- the second biological signal comprises first data and second data, the first data being values measured over a period of time and the second data being values specifying a feature of the manner used to measure the first data;
the method comprises a first step and a second step, the first step being a learning step and the second step being a learning and analyzing step, wherein
- the first step processes the first data and the second data of the first biological signals by using an abstraction model and a generalized model, wherein the abstraction model is forced to learn features for the prediction of the first data of the first biological signals by using a classifier, the features being independent of the second data, and wherein the generalized model uses the deviation between the first data of the first biological signals and their prediction to create a prediction of the third data of the first biological signals; and
- the second step processes the second biological signal by the abstraction model used in the first step to adapt the abstraction model and to transform the generalized model used in the first step into an individualized model to create a prediction of third data of the second biological signal, wherein the third data labelling at least one medical or technical state connected with the second biological signal.

## Description

The invention relates to a method for analyzing a biological signal. In particular, the invention relates to a method for analyzing an electrocardiogram (ECG) obtained by means of 1-3-lead electrocardiographs, wherein the analysis is performed by using ECGs obtained by means of 12-lead electrocardiographs.

The invention fits into the field of intelligent signal and image analysis. Known self-learning AI models (AI: artificial intelligence), trained on data sets with signals of a first modality and applied on data sets with signals of a second modality achieve no high prediction accuracy and no causal explanations in comparison to the application of those models on data sets with signals of the first modality. Modality describes the manner of how signals have been measured, and includes the regional origin, the type of signal derivation or the measurement device, among other things. In practice, it could be clinical datasets with biological signals where the datasets with signals of the first modality are, for example, 12-lead ECGs and the datasets with signals of the second modality are, for example, simplified biological signals of 3-lead patch ECGs. The term "prediction" describes the use of AI to derive values of a continuous range such as the systolic blood pressure from inputs such as the photoplethysmogram (PPG). For this purpose, regression models can be used. The term "prediction" can also describe the use of AI to derive discrete values or categories such as "atrial fibrillation" or "no atrial fibrillation" from inputs such as the ECG. For this purpose, classification models can be used.

In the field of deep learning applications in signal and image analysis, artificial neural networks are trained using a large amount of available data and analyzed for interpretability using so-called xAI (explainable AI) methods [2,3]. Depending on the available data, features to solve the prediction task are learned and, in some cases, very high accuracy is achieved.

Autoencoder networks are used for efficient knowledge representation. The aim is to extract a compressed feature vector from a set of data that does represent the input signal best. In the field of biological signal analysis, they are used for feature extraction and dimension reduction [4].

Transfer learning methods describe techniques in which pre-trained models are used for a specific prediction task and then applied to a related prediction task. During training for the related prediction task, the knowledge learned from the pre-trained model is used to increase the accuracy of the model in the related prediction task [5]. This is particularly used to improve specific prediction tasks with limited data sets.

The generalizability of models, in the sense of the ability of a model trained on a source domain to be transferable to a target domain, is referred to as domain generalization. Domain adaptation methods describe how preprocessing steps or model architectures can be modified [6] in order to achieve the generalizability of models by learning domain-independent features. The source and target domains should be coherent and describable to a certain extent.

Artificial neural networks achieve high prediction accuracies and are generally explainable using xAI methods. However, this only applies if the data sets for training and application are comparable, or the models have learned characteristics that are independent of domain-specific information of the two data sets (problem of domain generalization). In the field of biological signal analysis of the ECG, for example, large amounts of data from standard clinical derivations (12-lead ECGs) are available. However, new applications are aimed at mobile, non-clinical use with a minimalized setting (e.g. 3-lead ECG patch). These biological signals can be morphologically altered compared to standard ECG leads. The models, which were trained on 12- lead ECGs, then do not know the changed morphology of the 3-lead ECG patch signal and are therefore only partially usable on biological signals of a minimalized setting under impairment of the prediction accuracy and the validity and causality of explanations.

With methods of domain adaptation, information that is independent of the domain and prediction task may be lost (abstraction to generalization). However, this information in particular can be highly relevant if the generalized models are adapted to new individuals (e.g. patients) or fields of application (e.g. pathologies).

Models pretrained on a first prediction task and subsequently adapted to a second prediction task using transfer learning serve to solve the second prediction task more effectively compared to models that were trained directly on the second prediction task. However, domain differences, which exist in addition to potentially different prediction tasks, are not specifically considered or learned during pretraining. Thus, these models are highly specialized und individualized, but not generalizable and therefore limited in their transferability to unknown data.

This leads to the technical problem of how to learn comprehensible models in a generalized way and subsequently apply them to specific individualized problems with consideration of domain shifts, different prediction tasks and validity and causality of model explanations. Especially if limited data are available in the target domain to train the models.

The problem solved by the invention is the elimination of the drawbacks according to the current state of the art. In particular, a method for analyzing a biological signal is provided that achieves higher prediction accuracy.

This problem is solved by the features of claims 1 and 15. Practical developments of the inventions result from the features of the dependent claims.

According to the invention there is provided a method for analyzing a biological signal, wherein first biological signals measured in a first manner are used to analyze at least one second biological signal measured in a second manner, wherein
- the first biological signals comprise first data, second data and third data, the first data being values measured over a period of time, the second data being values specifying a feature of the manner used to measure the first data, and the third data labeling at least one medical or technical state assigned to the first biological signals; and
- the second biological signal comprises first data and second data, the first data being values measured over a period of time and the second data being values specifying a feature of the manner used to measure the first data;
the method comprises a first step and a second step, the first step being a learning step and the second step being a learning and analyzing step, wherein
- the first step processes the first data and the second data of the first biological signals by using an abstraction model and a generalized model, wherein the abstraction model is forced to learn features for the prediction of the first data of the first biological signals by using a classifier, the features being independent of the second data, and wherein the generalized model uses the deviation between the first data of the first biological signals and their prediction to create a prediction of the third data of the first biological signals; and
- the second step processes the second biological signal by the abstraction model used in the first step to adapt the abstraction model and to transform the generalized model used in the first step into an individualized model to create a prediction of third data of the second biological signal, wherein the third data labelling at least one medical or technical state connected with the second biological signal.

The invention is directed to the analysis of a biological signal. The biological signal to be analyzed is the second biological signal or, if two or more second biological signals are used, one of the second biological signals. Each second biological signal is additionally used to retrain the trained abstraction model. The accuracy of the analysis increases with the number of second biological signals, which have been analyzed before in the second step. This means that it is sufficient to retrain the trained abstraction model by using exactly one second biological signal, it is however preferred to retrain the trained abstraction model by using more than one second biological signal. This means in other words, that the invention allows to analyze a second biological signal by using the first biological signals in the first step and, optionally and preferably, by using all second biological signals which have been analyzed before in the second step. In a preferred embodiment, the method according to the invention is a method for analyzing a second biological signal, wherein first biological signals measured in a first manner are used to analyze second biological signals measured in a second manner. It is preferred to use two or more second biological signals to retrain the trained abstractions model in the second step.

The present invention achieves higher prediction accuracy on the analyzed second biological signal compared to the state of the art. In particular, the present invention achieves higher prediction accuracy on biological signals of another modality compared to the biological signals which have mainly been used for training. The method according to invention uses the first biological signals measured in a first manner to analyze a second biological signal measured in a second manner.

The first step processes the first biological signals to train the abstraction model and the generalized model to create a trained abstraction model and a trained generalized model. Therefore, the first step is a learning step. The second step uses the second biological signal to retrain the trained abstraction model from the first step and to analyze the second biological signal. The result of the analysis is the prediction of at least one medical or technical state connected with the second biological signal, i.e. the third data.

The first biological signals are used to train an abstraction model and a generalized model. The training transforms the abstraction model into a trained abstraction model and the generalized model into a trained generalized model. The trained generalized model processes the second biological signal. In this way, the trained generalized model is transformed into an individual model. The trained abstraction model processes the second biological signal and is additionally retrained by using said second biological signal. In this way, the trained abstraction model is adapted.

The term "biological signal" relates to a bioelectrical or non-bioelectrical time-varying signal in living beings that can be measured and monitored continually. A bioelectrical time-varying signal can be the change in electric current produced by the sum of an electrical potential difference across a specialized tissue, organ or cell system. Examples of biological signals of different electrical sources are ECGs, electroencephalograms (EEG), electromyograms (EMG), electrooculograms (EOG), electroretino-grams (ERG), electrogastrograms (EGG), galvanic skin responses (GSR) and electro-dermal activities (EDA). A biological time-varying signal can also be the change of a non-electric current. Examples of biological signals of different non-electrical sources are blood pressure (BP), which is the pressure of circulating blood against the walls of blood vessels, the PPG, which is an optical measure for blood volume changes, or respiration, which can be measured as the chest expansion. Preferably, biological signals are recorded and digitalized for further biological signal processing.

Preferably, the first biological signals are biological signals from a kind of source and the second biological signal is a biological signal from the same kind of source. The source can be a human or animal organ, a human or animal organ system, a human or animal tissue or a human or animal cell system. Preferably, the source is a human or animal organ or a human or animal organ system. A preferred human or animal organ is the heart or brain. A preferred human or animal organ system is the cardiovascular system.

The same type of biological signals can be measured in different manners. For example, ECGs may be measured by using technically different devices. A 12-lead electrocardiograph uses ten electrodes to measure twelve ECG leads. The biological signals of the twelve ECG leads are called 12-lead ECG. A 3-lead electrocardiograph uses three electrodes to measure three ECG leads. The biological signals of the three ECG leads are called 3-lead ECG. A 3-lead electrocardiograph differs from the 12-lead electrocardiograph in the number of electrodes and the position of the electrodes on a patient's body. Although biological signals measured in a first manner, for example measured by using a 12-lead electrocardiograph, and biological signals measured in a second manner, for example measured by using a 3-lead electrocardiograph, are measured in the different manners, both manners use the same basic principle to measure the electrical activity of the heart, namely the measurement by using electrodes. A 1-lead electrocardiograph uses only two electrodes to measure the electrical activity of the heart. Electrocardiographs with two or three electrodes that are attached directly to the skin of the patient's thorax are also referred to as ECG patches.

The term "manner" describes the method used to measure a biological signal. In one embodiment, the term "manner" describes the regional origin of the biological signal, the type of signal derivation and/or the measurement device. Preferably, the first manner and second manner measure biological signals of the same kind of organ, organ system, tissue or cell system, but by using different methods. The methods are different if the number of electrodes, the number of leads, or the placement of the electrodes is different. For example, a standard 12-lead electrocardiograph and a 3-lead electrocardiograph measure biological signals of the heart, but the manner of a 12-lead electrocardiograph differs from the manner of a 3-lead ECG patch in the number of electrodes, the number of leads, and the placement of the electrodes. In medical technology (MedTech), the term "modality" is often used to describe the method used to measure a biological signal. In the present application, the term "manner" and "modality" are used synonymously.

Preferably, the first biological signals are ECGs measured by a device having a first number of leads and the second biological signal is an ECG measured by a device having a different number of leads. More preferably, the first biological signals are ECGs measured by a 12-lead electrocardiograph and the second biological signal is an ECG measured by a 3-lead electrocardiograph, a 2-lead electrocardiograph or a 1-lead electrocardiograph. Most preferably, the second biological signal is an ECG measured by a 3-lead electrocardiograph.

The first biological signals comprise first data, second data and third data. In contrast, the second biological signal comprises first data and second data, but no third data. The method according to the invention enables the prediction of the third data of the second biological signal by using the first data, the second data and the third data of the first biological signals. The first data, the second data and the third data of the first biological signals are from one or more data sets. Each data set is a collection of a large quantity of first biological signals.

The first data of a biological signal are values measured over a period of time. Examples of such values are the electrical activities of the heart, the electrical activities of the brain, the electrical activities of a skeletal muscle, the corneo-retinal standing potential that exists between the front and the back of an eye, electrical responses of cell types in the retina, electrical signal generated by the movement of the smooth muscle of the stomach, electrical activities of the skin, or changed light absorption characteristics of human or animal tissue due to changes in the volume or properties of blood in the tissue. Preferably, the first biological signals and the second biological signal relate to values from the same kind of organ or the same kind of tissue. For example, if the first biological signals comprise first data relating to electrical activities of the heart, the second biological signal also comprises first data relating to electrical activities of the heart. The first data are often referred to as "signals" because they are the measured values of an electrical signal.

The second data are values specifying a feature of the manner used to measure the first data. The feature enables a classification of the manner. Examples of such features are the position of the electrode on the patient's body, the kind of organ or tissue, and the kind of device used for measuring the first data. For example, the second data of an ECG measured by using a 12-lead electrocardiograph are the lead and the kind of the device, namely a 12-lead electrocardiograph. The second data of an ECG measured by using a 3-lead electrocardiograph are the lead and the kind of the device, namely a 3-lead electrocardiograph. This means that the first data can be classified by the indication "fifth lead of a 12-lead electrocardiograph" or "second lead of a 3-lead electrocardiograph". In the following, the second data are also referred to as domain. For example, the term "domain classifier" relates to a classifier used for classifying the domain of the first biological signal.

The third data labeling at least one medical or technical state assigned to the first biological signals. The first biological signals are classified by the indication of a medical or technical state. The classifications are labels to the first biological signals and saved together with the first data and the second data of a first biological signal in one or multiple data sets. The labels were assigned to the first biological signals by a human being, for example a physician. The labels are also referred to as "prediction labels" because the assignment of a medical or technical state is a prediction of the first data.

The second biological signal does not comprise third data. The method according to the invention is used to predict third data. Preferably, the third data are predicted together with a value of probability and/or an explanation. The value of probability shows the decision security of the model in predicting the third data, that is, the value of probability estimates the quality of the prediction. The explanation can be an indication disclosing a feature of the first data of the second biological signal, that was decisive for the prediction of the medical or technical state. The medical state can describe a disease like atrial fibrillation, non-atrial fibrillation or the presence or absence of a pathologic feature. The technical state can describe the presence or absence of a pathologic feature or the useability or the lack of useability of the first data of the second biological signal.

In relation to an ECG, the first data are the electrical activities measured on a patient's body, these data are also referred to as "signals" in the following examples; the second data is the name of the respective lead through which the first data were received, these data is also referred to as "domains" in the following examples; and the third data is a label describing a medical and/or technical state connected with the first data, for example atrial fibrillation, these data is also referred to as "label" or "prediction label" in the following examples.

The term "prediction" describes the use of AI to derive values of a continuous range such as the systolic blood pressure from inputs such as the PPG. For this purpose, regression models can be used. The term "prediction" can also describe the use of AI to derive discrete values or categories such as "atrial fibrillation" or "no atrial fibrillation" from inputs such as the ECG. For this purpose, classification models can be used. The invention covers both descriptions of "prediction".

The term "abstraction model" relates to an artificial neural network. The first step is a general learning step. The second step is an individualized learning and analyzing step. In the first step, the artificial neural network is used to generate a prediction of first data of the first biological signals. In the second step, the artificial neural network is used to generate a prediction of first data of the second biological signal. The abstraction model can be an autoencoder. In the first step, the abstraction model comprises a classifier. The classifier is an algorithm used to generate second data to classify the first data of the first biological signals. The classifier is trained to unsuccessfully deduce second data from the prediction of the first data of the first biological signals. Provided that the second data relate the domain of a biological signal, the abstraction model can be considered to be a domain adversarial neural network. In the second step, the abstraction model does not comprise a classifier to generate second data to classify the first data of second biological signal.

In the first step, the abstraction model is forced to learn features for the prediction of the first data of the first biological signals that are independent from the second data of the first biological signals by using the classifier. In the second step, the trained abstraction model is forced to learn features for the prediction of the first data of the second biological signal without using a classifier. The features depend on the second data of the second biological signal. This means, that the abstraction model learns features in the first step, which are independent from the second data of the first biological signals, and that the learned abstraction model learns features, which are specific for the second data of the second biological signal. The term "feature" relates to characteristics or properties of the input data that are used by the abstraction model or the trained abstraction model to make predictions.

The term "generalized model" relates to a further artificial neural network with multiple layers, at least a first and a last layer. An artificial neural network always consists of at least one input layer and at least one output layer. There are usually several hidden layers between the at least one input layer and the at least one output layer. Within these hidden layers, artificial neural networks extract features from the input data. These features are used for prediction in the final layer. In the first step, the artificial neural network is used to generate a prediction of third data of the first biological signals. In the second step, the artificial neural network is used to generate a prediction of third data of the second biological signal. The generalized model can contain a feature extractor. In the first step, the generalized model comprises a classifier. The classifier is an algorithm used to generate second data to classify the first data of first biological signals. The classifier is trained to unsuccessfully deduce second data from the prediction of the first data of the first biological signals. Provided that the second data relate to the domain of a biological signal, the generalized model can be considered to be a domain adversarial neural network. In the second step, the generalized model does not comprise a classifier to generate second data to classify the first data of the second biological signal.

Preferably, the generalized model uses an attention mask. In the first step, the attention mask is weighted with the reciprocal of the deviation between the first data of the first biological signals and the prediction of the first data of the first biological signals, that is generated by using the abstraction model. In the second step, the attention mask is weighted with the reciprocal of the deviation between the first data of the second biological signal and the prediction of the first data of the second biological signal, that is generated by using the trained abstraction model. Instead of an attention mask, an individualized feature vector from the abstraction model can also be passed to the generalized model.

In the first step, the generalized model further uses the first data, the second data, the third data and the deviation between the first data of the first biological signals and their prediction to create features used for the prediction of the third data of the first biological signals. In the second step, the trained generalized model further uses the first data of the second biological signal and the deviation between the first data of the second biological signal and their prediction to create features used for the prediction of the third data of the second biological signal. The term "feature" relates to characteristics or properties of the used first data that are used by the generalized model or the trained generalized model to make predictions.

Preferably, the first data of the second biological signal and the deviation between the first data of the second biological signal and their prediction are processed by the trained generalized model to transform the trained generalized model into the individualized model and to create a prediction of the third data of the second biological signal.

In the first step, an abstraction model for the prediction of the first data depending on the second data, for example the domain, and a generalized model for the prediction of the label depending on the second data are trained. However, the abstraction model is forced to learn domain-independent features for the prediction of the signal, i.e., the abstraction model is a domain adversarial neural network. The reciprocal of the deviation between the prediction of the original first data of the first biological signals is then entered into the generalized model as an attention mask, in order to create an adapted prediction of the third data, for example a label, based on prior knowledge of the generalized model. Instead of an attention mask, an individualized feature vector from the abstraction model can also be passed to the generalized model. Also, the generalized model can be forced by means of domain adversarial learning to learn domain-independent features, which are then weighted by the attention mask or offset against the passed feature vector. For example, an autoencoder architecture can be used as an abstraction model and a classification model with a classic deep learning architecture, e. g. a convolutional neural network (CNN), as a generalized model. Both architectures must be supplemented with a domain classifier to take into account the domain und thus to learn domain-independent features.

In the second step, the trained abstraction model that was used in the first step is used without the domain classifier. The aim here is to make a continuous adjustment based on the predictions and the incoming first data of the second biological signal by retraining the trained abstraction model. This step in turn adapts the trained abstraction model to the newly received data, i.e., the trained abstraction model is transformed into an individualized trained abstraction model. The resulting deviation between the individualized prediction of the first data of the second biological signal and the original first data of the second biological signal is then entered into the trained generalized model that was obtained in the first step. Said deviation is entered into the trained generalized model as an attention mask. If an adapted feature vector instead of an attention mask is entered within the first step, the feature vector is individualized in the second step and entered into the trained generalized model. In the second step, the trained generalized model is again used without the domain classifier and outputs a label prediction. Thus, the trained generalized model that was obtained in the first step is used without the domain classifier, an individualized prediction is possible, and an individualized model emerges without retraining the individualized model.

Using xAI methods, explanations of both models from the first step and the second step can be generated and projected onto the original second biological signal. These explanations, together with those of the classification in both steps, are results of the learning architecture.

The present invention creates a deep learning model by a learning architecture, which both learns generalized and individualized features and enables comprehensible tracing of the features. Linked to both, it allows to force models on a modality with a lot of available data to train a generalized model, which can subsequently be used for data from another modality or the same with fewer amount of available data. This is done with the connection by the abstraction model.

The added value of the present invention is achieved by a special learning architecture of self-learning algorithms from the field of deep learning. It enables learning from a large amount of data, consisting of the first biological signals from a first modality, as well as the generalization for possibly limited amount of data, consisting of a second biological signal from a second modality. Through subsequent individualized learning, which the AI models carry out independently through the present invention, an adaptation is made, without the models having to be actively adapted to the data of the second modality. This makes it possible to adapt the overall model to additional application areas and classification or prediction tasks for which insufficient training data are available. Every step in the model, that is generalization and individualization, can be supplemented in detail with methods from explainability. Thus, both explanations of generalizability and individualization can be provided and used for interpretation. The individualization creates new fields of application on the basis of trained generalized models. The individualization is the basis for a comprehensible (by explanations) model architecture for knowledge extraction from limited available knowledge bases. This can be used in particular in the clinical context to use generalized models in the sense of individualized medicine. The architecture contributes to meet the interpretability requirement of Article 13 of the EU AI Act [1].

The combined model that is described in DE 10 2023 118 246 A1 and consists of a long-term and a short-term model, can be integrated and used to detect previously unknown phenomena and characteristics for little existing signals of a related modality, e.g. an ECG patch or a rare pathology, and additionally offers an individualization to the individual, e.g. a patient. Both applications have an enormous potential, on its own and together. In DE 10 2023 118 246 A1, the focus is placed on an interpretable representation of explanations of self-learning models of AI with the distinction in two categories, in the case of rhythm and morphology, by the back projection of the explanations into the output signal.

According to the invention there is further provided a measuring device for recording an ECG, the device comprising
- at least one sensor, which in case of the ECG comprises at least two electrodes, for measuring at least one second biological signal, the second biological signal comprises first data and second data, the first data being values measured over a period of time and the second data being values specifying the manner used to measure the first data;
- a memory storing a trained abstraction model of an artificial neural network and an individualized model of an artificial neural network, the artificial neural networks being trained to create a prediction of third data of the second biological signal, wherein the third data labelling at least one medical or technical state connected with the second biological signal; and
- a processing unit comprising one or more processors configured to
   - receive the second biological signal; and
   - execute a neural network engine, the neural network engine implementing the neural networks using the trained abstraction model and the individualized model for inferring one or more output based on inputs, the one or more output providing a prediction of the third data.

Details of the trained abstraction model and the individualized model have already been described in connection with the method according to the invention. Reference is made to this description.

The term "neural network engine" relates to a software or hardware framework that builds, trains, and runs artificial neural networks, enabling them to process data and make predictions.

The invention provides a novel learning architecture of a deep learning network, consisting of a generalized learning step and an individualized learning and analyzing step. The generalized learning is performed by the first step of the method according to the invention. The individualized learning is performed by the second step of the method according to the invention. The new learning architecture enables the adaptation of a generalized model trained with first biological signals that were measured in a first manner to an individualized model to predict features of the second biological signal that was measured in a second manner. Furthermore, generalized features are learned, individually adapted and with methods of xAI comprehensively visualized. The learned features can thus ultimately be used for both generalized and individualized evaluation, e.g. diagnostic support for individualized medicine.

There are already large clinical data sets for standard clinical modalities (e.g. 12-lead ECG), which can be used to train AI models and achieve high classification performance and valid explanations for accurate labels. However, there are also rare pathologies for which less data are available. In this case, a basic knowledge of characteristics from other data sets can be learned through the learning architecture and later individualized to a smaller, rarer data set. This has the potential to learn about previously unknown phenomena and their characteristics and to represent them in a comprehensible way in the signal.

Furthermore, a generalized model can be learned on the basis of clinical data sets of standard modalities, which learns domain-independent features and thus can be applied and individualized to comparable modalities of another domain. This is absolutely necessary, because, for example, in biological signal analysis, different ECG leads have morphologically different characteristics.

The signaling is also dependent on other factors, such as the individual orientation of the heart and deviations in the attachment of the modality to the subject. Therefore, an individualization of the models is necessary in order to provide accurate biological signal analyses and, if necessary, diagnostic support.

Both applications are possible individually and combined in the invention, which has not been possible until now.

The presented architecture can be installed as a trustworthy, interpretable component in automated medical diagnostic systems (e.g. ECG patches). This will achieve the objectives of the EU AI Act on transparent AI in risk areas such as medicine at an unprecedented level.

In the following, the invention is explained in more detail with the help of examples not intended to limit the invention with respect to the drawings. Here
- Fig. 1: represents a first embodiment of the learning architecture used for performing an example of the method according to the invention;
- Fig. 2: represents a detail of the architecture shown in Fig. 1 to illustrate the first step;

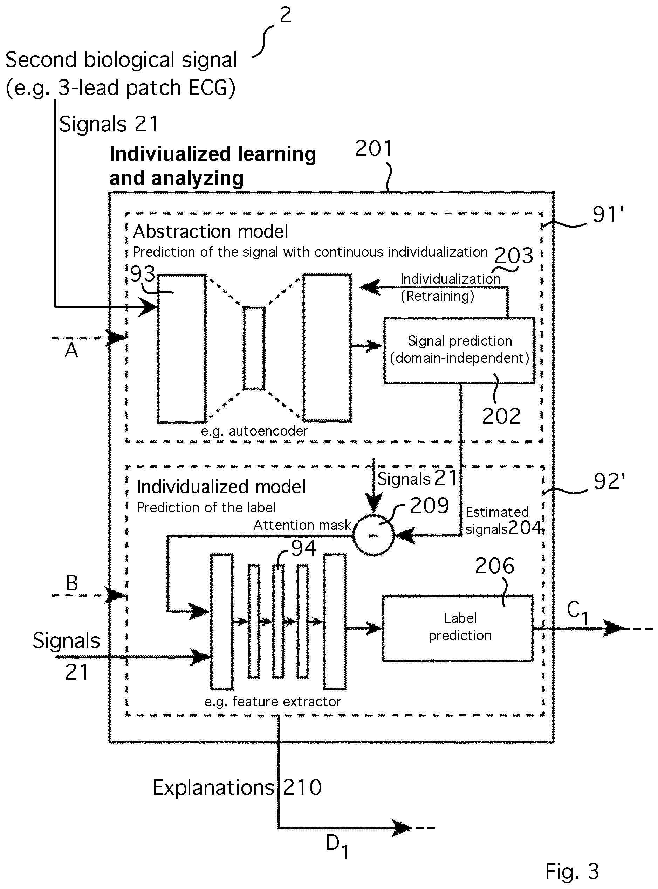

- Fig. 3: represents a further detail of the architecture shown in Fig. 1 to illustrate the second step; and
- Fig. 4: represents a second embodiment of the architecture used for performing an example of the method according to the invention.

### Example 1

In this example, the invention provides comprehensible and high-performance classification models for ECG signals, which are recorded using mobile ECG patches.

Mobile electrocardiographs usually have 1-3 ECG leads compared to the classic 12-lead electrocardiograph. Such mobile electrocardiographs are also called ECG patches. Many large clinical data sets (e.g. on PhysioNet.org) with gold standard labels are available for the 12-lead electrocardiographs. In contrast, very few gold-standard labeled data sets for mobile electrocardiographs are available so far. Therefore, in this example, the signal of modality 1 is to represent an ECG obtained by using a 12-lead electrocardiograph, and the signal of modality 2 is to represent the 3-lead patch ECG. In this example, the aim is the accurate detection of atrial fibrillation in the signal of modality 2.

The 12-lead ECG as well as the 1-3-lead ECG can have a varying sampling rate. However, it is recommended to use at least 500 Hz for the extraction of morphology features in ECGs. In case of different sampling rates, resampling can be applied to align the sampling rates of both modalities to each other and to the recommended sampling rate of 500 Hz. Typical ECG preprocessing steps such as a high-pass filter with a cutoff frequency of 0.3 Hz or filters for noise reduction or interference elimination are recommended.

In the following, an implementation of the learning architecture for the detection of atrial fibrillation from 3-lead patch ECGs is presented.

### Data and pre-processing

### a) First biological signals

Electrical signals of the heart have been measured with a 12-lead electrocardiograph. These electrical signals are the first biological signals. The term "12-lead ECG" refers to the manner in which the first biological signals have been measured. This manner is the first manner. To measure a 12-lead ECG, an electrocardiograph having 10 electrodes is used, wherein the ten electrodes are placed on the patient's limbs and on the surface of the chest. The electrical potential of the heart is then measured from twelve different angles and is recorded over a period of time. The twelve different angles are the leads of the 12-lead ECG.

The biological signals obtained with the 12-lead ECG comprise first data, second data and third data. The first data are electrical potentials of the heart measured from the twelve different angles and recorded over a period of time. The second data classify the measurement of the first data by 12-lead ECG and the corresponding lead of 12-lead ECG via which the first data have been obtained. Data for the same period of time are obtained via each of the twelve leads. The first data of all twelve leads together form the first data of the first biological signals.

The third data refer to the medical state that is associated with the first data. In this example, the third data are the label "atrial fibrillation" or the label "no atrial fibrillation". The prediction of the third data of the second biological signal 2 is the prediction task.

The first biological signals originate from a clinical data set. The clinical data set comprises a large number of first biological signals, each including their first, second and third data.

In the following, the first data of the 12-lead ECG are referred to as "signals", the second data as "domains" and the third data as "label" or "prediction label". In the first step, the first, second and third data, thus signals, domains and label, are used.

### b) Second biological signal

Electrical signals of the heart have been measured as a 3-lead ECG with a mobile electrocardiograph. These electrical signals are the second biological signal. The term "3-lead ECG" refers to the manner in which the second biological signal has been measured. This manner is the second manner. To measure a 3-lead ECG, a device having 3 electrodes is used, wherein the three electrodes are placed on the surface of the patient's chest. The electrical potentials of the heart are then measured from three different angles and are recorded over a period of time. The three different angles are the leads of the 3-lead ECG.

The biological signals obtained with the 3-lead electrocardiograph comprise first data and second data. The first data are electrical potentials of the heart measured from the three different angles and recorded over a period of time. The second data classify the measurement of the first data by 3-lead electrocardiograph and the corresponding lead of the 3-lead ECG, via which the first data have been obtained. These second data differ from the second data of the first biological signals. Data for the same period of time are obtained via each of the three leads. The first data of all three leads together form the first data of the second biological signal.

It is not absolutely necessary, that the second biological signal contains second data, in particular information about the ECG lead.

The biological signals obtained with the 3-lead electrocardiograph do not contain third data, that refer to the medical state that is associated with the first data. With the embodiment of the method according to the invention shown in this example, third data shall be predicted that characterize the medical state that is associated with the second biological signal. In this example, the biological signals obtained with the 3-lead ECG are analyzed to predict, whether the second biological signal shall be labeled with the label "atrial fibrillation" or the label "no atrial fibrillation".

In the following, the first data of the 3-lead ECG are referred to as "signals" and the second data as "domains". In the second step, the first data and, optionally, the second data of the second biological signal, thus its signals and domains, are used to predict its third data, i.e. the label, that characterizes the medical state that is associated with the second biological signal.

### Learning architecture

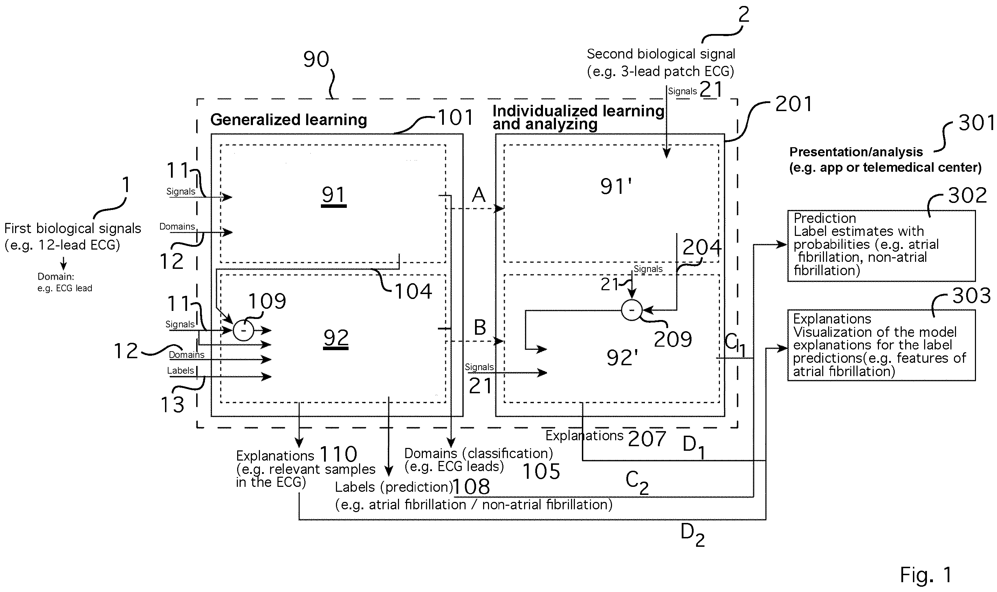

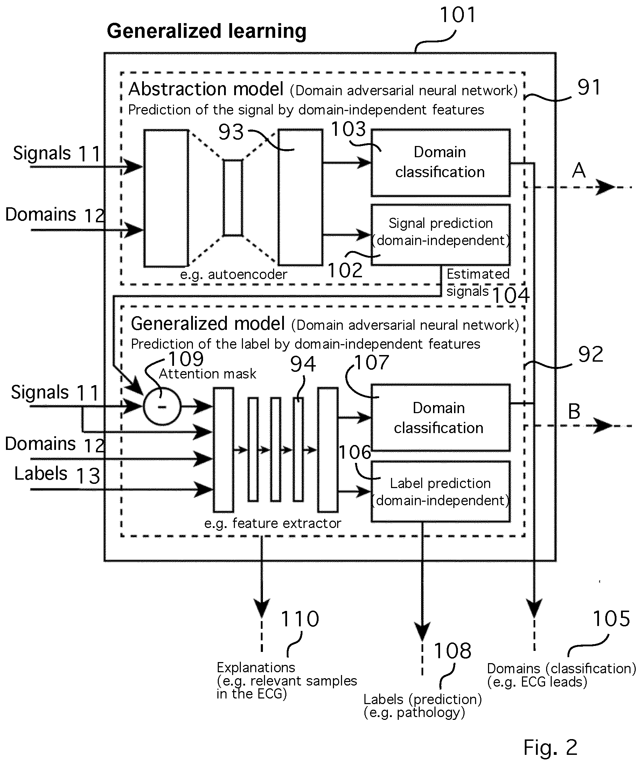

The learning architecture 90 comprises an abstraction model 91 and generalized model 92 (see Fig. 1). The abstraction model 91 is an artificial neural network, in this example a domain adversarial neural network. In the first step, the abstraction model 91 comprises an autoencoder 93 with domain classifier (see Fig. 2). The generalized model 92 is an artificial neural network, in this example a domain adversarial neural network. In the first step, the generalized model 92 comprises a feature extractor 94 with domain classifier (see Fig. 2).

The abstraction model 91 and the generalized model 92 are used in the first step 101. The results of the first step are a trained abstraction model 91' and a trained generalized model 92'. After completing the first step 101, the trained abstraction model 91' and the trained generalized model 92' are transferred to the second step 201. The domain classifier is removed from the trained abstraction model 91'. The domain classifier is also removed from the trained generalized model 92'.

Since the trained generalized model 92' makes individualized predictions und thus delivers individual explanations, this trained generalized model 92' is also called "individualized model" in the second step. The trained generalized model 92' is transformed into the individualized model by using the second biological signal.

In this example, the first step runs on a high-performance computing unit with available clinical data sets, consisting of 12-lead ECGs labelled with third data.

The second step can run on a high-performance computing unit or a reduced computing unit of an ECG patch with incoming ECG recordings. The second step is carried out using one or multiple 3-lead patch ECGs.

### First step

In the first step, the 12-lead ECGs 1 with signals 11, the domains 12, i.e. the name of the corresponding ECG leads, and the prediction labels 13, i.e. atrial fibrillation or non-atrial fibrillation, are entered into learning architecture 90 (see Fig. 1). The 12-lead ECGs 1 are first biological signals of a first modality. They are recordings of the first modality.

The learning architecture 90 comprises an abstraction model 91 and generalized model 92. The abstraction model 91 is an artificial neural network, in this example a domain adversarial neural network. In the first step, the abstraction model 91 comprises an autoencoder 93 with domain classifier (see Fig. 2). The generalized model 92 is an artificial neural network, in this example a domain adversarial neural network. The abstraction model 91 and the generalized model 92 are used in the first step 101.

The first step 101 is a training phase enabling generalized learning. The abstraction model 91 is trained to predict the signals 11 independent from the domains 12 of ECG 1, i.e. the lead. This is implemented as the autoencoder 93 with domain classifier. Inputs are the signals 11 of ECG 1 and the domains 12 of the respective ECG leads. Outputs are a prediction 102 of the signals 11 of ECG 1 and a classification 103 of the domains 12 of the ECG 1. The prediction 102 of the signals 11 of the ECG 1 in the following is also referred to as "estimated signals 104". The prediction 102 of the signals 11 is domain-independent. The classification 103 of the domains 12 of the ECG 1 is an estimation of the ECG lead and thus a domain classification. The domain classifier is however set up so that it cannot classify the signals 11 to determine the domains of signals 11. To achieve this, the accuracy of the domain classification 103 is minimized during training. The predicted domains 105, that is the classification 103 of the estimated signals 104, are outputted. The output of the predicted domains 105 make it possible to check that the classification failed, which means that the predicted domains 105 differ from the actual domains 12.

Using the autoencoder 93, the signals 11 from ECG 1 are reduced to a code (encoder) and then the original signals 11 are estimated from the code (decoder). The code can be understood as a learned feature space that contains all the necessary information to learn domain-independent features by the domain classifier. The domain classifier is therefore learned in such a way that it cannot classify the domain, this means that the domain is incorrectly classified.

The deviation of the thus obtained estimated signals 104 from the original signals 11 is entered into the generalized model 92 for the prediction 106 of the prediction labels 13 depending on the domains 12. The reciprocal of the deviation is entered into the generalized model 92 as an attention mask 109. Due to the use of the domain classifier, the label prediction 106 is domain independent. During training the accuracy of domain classification 107 is minimized. This means that features, that are learned for label prediction 106, are however independent of the domains 12 used for domain classification 107. Instead of or additionally, however, the learned code of the autoencoder can also be transferred to the feature space of the generalized model 92.

The generalized model 92 can be implemented as a domain adversarial neural network with feature extractor 94. The domain adversarial neural network with feature extractor can be a deep neural network. Again, the domain classification task is forced for misclassification. The attention mask 109, the signals 11, the domains 12, i.e. the ECG leads, and the prediction labels 13 are inputs.

Outputs are a prediction 106 of the label 13 of the ECG 1, a classification 107 of the domains 12 of the ECG 1 and explanations 110. The label prediction 106 is domain-independent. The domain classification 107 is an estimation of the ECG lead. The domain classification 107 is however set up so that it cannot classify the signals 11 to determine the domains 12 of ECG 1. The predicted domain classification 105, that is the failed classification, is outputted. The output of the predicted domain classification 105 makes it possible to check that the classification failed. The explanations allow for example an identification of relevant samples in the ECG 1 for the label prediction 106. This makes the prediction more comprehensible and therefore the AI more trustworthy. Deep Taylor Decomposition is used as the explanation method in this example. The explanation method is a method of xAI. The predicted labels 108 and the explanations 110 are outputted.

After completing the first step 101, the abstraction model 91 and the generalized model 92 are transferred to the second step 102 (arrows A and B). Therefore, in both models 91, 92 the domain classifiers will be removed. Since both models are forced to misclassify the domains 12, this is elemental for the individualization.

The first step relies on the collection of a large quantity of first biological signals, which are used to train the abstraction model 91 and the generalized model 92. The first step is a training phase. The results of the training phase are a trained abstraction model 91' and a trained generalized model 92', which are used in a second step to generate output. Output are the label prediction 206, i.e. the prediction of the medical or technical state that could be connected with the second biological signal, and explanations 210. The label prediction 206 is the result of the analysis of the second biological signal 2.

### Second step

In the second step 201 an operational phase enables individualized learning and analyzing. The second step 201, the trained abstraction model 91' and the trained generalized model 92' are used without the domain classifier.

In the second step, the 3-lead ECG 2 with signals 21 is entered into learning architecture 90 (see Fig. 1). The 3-lead ECG 2 is a second biological signal of a second modality. Domains, i.e., the name of the corresponding ECG leads, and a prediction label are not entered.

As an input, the signals 21 of the ECG 2 are entered into the trained abstraction model 91', in this example the autoencoder 93. The autoencoder 93 is the same as that used in the first step. When the input signals 21 are entered into the autoencoder 93, a prediction 202 of the signals 21 is made on the basis of the trained abstraction model 91'. The prediction 202 of the signals 21 of the ECG 2 in the following is also referred to as "estimated signals 204".

The prediction 202 is used to individualize the trained abstraction model 91'. This individualization 203 is used to retrain the trained abstraction model 91'. The retraining is performed each time signals 21 are inputted into the autoencoder 93. In this way, the autoencoder 93 is continuously adapted. Thus, in the second step, an individualization of the autoencoder 93 and the prediction 202 of the signals 21 is done. The prediction 202 of the signals 21 is the output of the trained abstraction model 91'.

The deviation of the thus obtained estimated signals 204 from the original signals 21 is entered into the generalized model 92' for the prediction 206 of the prediction label. Said the reciprocal of the deviation is entered into the generalized model 92' as an attention mask 209. Consistent to the trained abstraction model 91', the deviation from estimated signals 204 and the original signals 21, or in addition, the learned code of the autoencoder 93 can be transferred into the feature space of the trained generalized model 92'. This trained generalized model 92' uses the input data, that is the original signals 21 and the deviation estimated signals 204 from the original signals 21 for a classification and a prediction 206 of a prediction label of the 3-lead ECG 2. The prediction label are third data of the 3-lead ECG 2.

Apart from the prediction 206, explanations 210 are outputted. The explanations allow for example a global explanation of the trained generalized model 92' and/or the identification of relevant samples in the 3-lead ECG 2 for an individual label prediction 206. This makes the prediction more comprehensible and therefore the AI more trustworthy. Deep Taylor Decomposition is used as the explanation method in this example. The explanation method is a method of xAI.

### Presentation of the results

The prediction 206 of the prediction label is presented, for example by a presentation 301 on a display. The display can be a part of a computer. A computer application can be used for the presentation 301. The presentation 301 can take place in a telemedicine center.

The presentation 301 comprises a presentation 302 of the prediction 206 of the prediction label (arrow C1). The prediction 206 can be accompanied by an indication of the probability that the prediction 206 is correct, which is represented for example by the model security for decision-making. The presentation 302 can also comprise the prediction 108 of a prediction label generated in the first step 101 (arrow C2). In the example, the prediction label is "atrial fibrillation" or "non-atrial fibrillation".

The presentation 301 can further comprise a presentation 303 of the explanation 110 (arrow D2) and the explanation 210 (arrow D1). These explanations 110, 210 are a visualization of the sample relevance to the generalized model 92, 92' used for domain classification 107 or label prediction 106, 206.

The output of the models of the first step and the second step consist of the label predictions (e.g. atrial fibrillation, non-atrial fibrillation) and the model explanations, which are projected on the original signal inputs. This means explanations from the first step to the 12-lead ECG and from the second step to the 3-lead patch ECG.

### Example 2

Example 2 corresponds to Example 1 apart from using third biological signals 3 additionally to the first biological signals. The third biological signals are biological signals of the heart and were measured with a 3-lead electrocardiograph. The term "3-lead ECG" refers to the manner in which the third biological signals was measured. This manner corresponds to the second manner. To measure a 3-lead ECG, a device having 3 electrodes is used, wherein the three electrodes are placed on the surface of the patient's thorax. The electrical potentials of the heart are then measured from three different angles and are recorded over a period of time. The three different angles are the leads of the 3-lead electrocardiograph.

The third biological signals obtained with the 3-lead electrocardiograph comprise first data, second data and third data. The first data are electrical potentials of the heart measured from the three different angles and recorded over a period of time. The second data identify the first data via a 3-lead electrocardiograph and the corresponding lead of 3-lead electrocardiograph, via which the first data have been obtained. Via each of the three leads data are obtained for the same period of time. The first data of all three leads together form the first data of the third biological signals. The third data refer to the medical state that is associated with the first data. In this example, the third data are the label "atrial fibrillation" or the label "no atrial fibrillation".

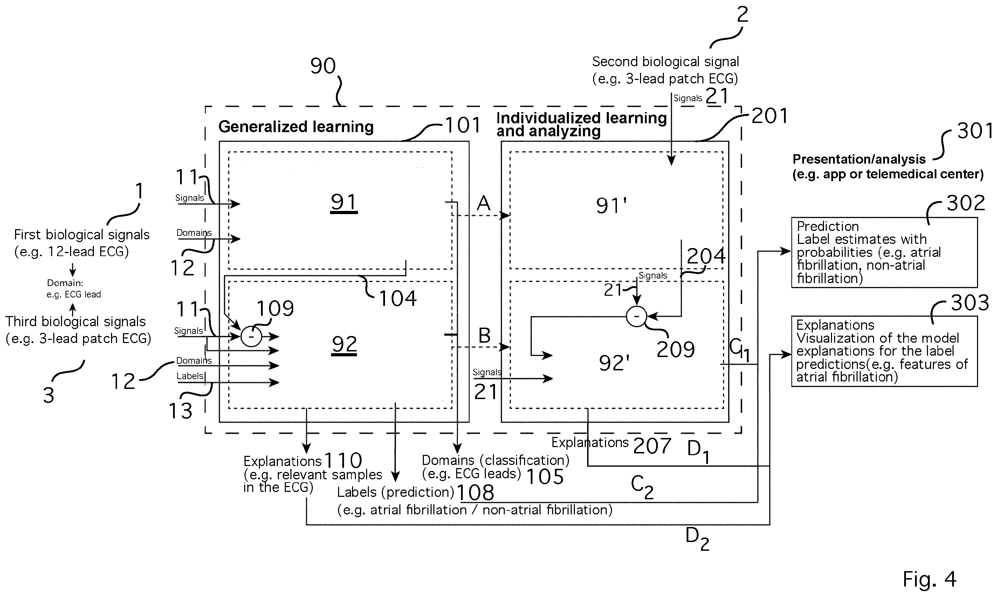

The third biological signals 3 (see Fig. 4) are from a clinical data set. The clinical data set comprises a large number of third biological signals 3, each including their first, second and third data.

The third biological signals 3 are used in the same manner as the first biological signals 1 in the first step to train an abstraction model 91 and a generalized model 92.

### References

[1] European Parliament, Council of the European Union. (2024). Regulation (EU) 2024/1689 of the European Parliament and of the Council of 13 June 2024 laying down harmonised rules on artificial intelligence and amending Regulations (EC) No 300/2008, (EU) No 167/2013, (EU) No 168/2013, (EU) 2018/858, (EU) 2018/1139 and (EU) 2019/2144 and Directives 2014/90/EU, (EU) 2016/797 and (EU) 2020/1828 (Artificial Intelligence Act) (Text with EEA relevance). Official Journal of the European Union. http://data.europa.eu/eli/reg/2024/1689/oj
[2] M. Goettling, A. Hammer, H. Malberg, and M. Schmidt, "xECGArch: a trustworthy deep learning architecture for interpretable ECG analysis considering short-term and long-term features," Sci Rep, vol. 14, no. 1, p. 13122, Jun. 2024, doi: 10.1038/s41598-024-63656-x.
[3] J. Vielhaben et al., "Explainable ai for time series via virtual inspection layers," Pattern Recognition 150 (2024): 110309.
[4] U. Lomoio et al., "A convolutional autoencoder framework for ECG signal analysis," Heliyon, vol. 11, no. 2, p. e41517, Jan. 2025, doi: 10.1016/j.heliyon.2024.e41517.
[5] J. Yosinski, J. Clune, Y. Bengio, and H. Lipson, "How transferable are features in deep neural networks?," in Proceedings of the 28th International Conference on Neural Information Processing Systems - Volume 2, in NIPS' 14, vol. 2. Cambridge, MA, USA: MIT Press, Dec. 2014, pp. 3320-3328.
[6] Y. Ganin et al., "Domain-Adversarial Training of Neural Networks," in Domain Adaptation in Computer Vision Applications, G. Csurka, Ed., Cham: Springer International Publishing, 2017, pp. 189-209. doi: 10.1007/978-3-319-58347-1_10.

## Claims

1. A method for analyzing a biological signal, wherein first biological signals measured in a first manner are used to analyze at least one second biological signal measured in a second manner, wherein
- the first biological signals comprise first data, second data and third data, the first data being values measured over a period of time, the second data being values specifying a feature of the manner used to measure the first data, and the third data labeling at least one medical or technical state assigned to the first biological signals; and
- the second biological signal comprises first data and second data, the first data being values measured over a period of time and the second data being values specifying a feature of the manner used to measure the first data;
the method comprises a first step and a second step, the first step being a learning step and the second step being a learning and analyzing step, wherein
- the first step processes the first data and the second data of the first biological signals by using an abstraction model and a generalized model, wherein the abstraction model is forced to learn features for the prediction of the first data of first biological signals by using a classifier, the features being independent of the second data, and wherein the generalized model uses the deviation between the first data of the first biological signals and their prediction to create a prediction of the third data of the first biological signals; and
- the second step processes the second biological signal by the abstraction model used in the first step to adapt the abstraction model and to transform the generalized model used in the first step into an individualized model to create a prediction of third data of the second biological signal, wherein the third data labelling at least one medical or technical state connected with the second biological signal.

2. The method according to claim 1, wherein the abstraction model is an autoencoder.

3. The method according to claim 1 or claim 2, wherein the generalized model is an artificial neural network.

4. The method according to claim 2 or claim 3, wherein the abstraction model comprises a classifier in the first step, wherein the classifier is trained to unsuccessfully deduce second data from the prediction of first data of the first biological signals.

5. The method according to any one of the preceding claims, wherein the generalized model uses an attention mask, wherein the attention mask is weighted with the reciprocal of the deviation between the first data of the first biological signals and their prediction.

6. The method according to any one of the preceding claims, wherein in the first step the generalized model further uses the first data of the first biological signals, the second data of the first biological signals, the third data of the first biological signals and the deviation between the first data of the first biological signals and its prediction to create features used for the prediction of the third data of the first biological signals.

7. The method according to claim 6, wherein the generalized model comprises a classifier in the first step, wherein the classifier is trained to unsuccessfully deduce second data from the features used for the prediction of the third data of the first biological signals.

8. The method according to any one of the preceding claims, wherein the second step comprises the use of the abstraction model for the prediction of the first data of the second biological signal.

9. The method according to claim 8, wherein the second step uses the deviation between the first data of the second biological signal and their prediction to create the prediction of the third data of the second biological signal.

10. The method according to claim 9, wherein the first data of the second biological signal and the deviation between the first data of the second biological signal and their prediction are processed by the generalized model to transform the generalized model into the individualized model and to create a prediction of the third data of the second biological signal.

11. The method according to any one of the preceding claims, wherein the first biological signals are biological signals from a kind of source and wherein the second biological signal is a biological signal from the same kind of source.

12. The method according to any one of the preceding claims, wherein the kind of source is a human or animal organ or a human or animal organ system.

13. The method according to any one of the preceding claims, wherein the human or animal organ is the heart or brain or wherein the human or animal organ system is the cardiovascular system.

14. The method according to any one of the preceding claims, wherein the first biological signals are electrocardiograms measured by a device having a first number of leads and the second biological signal is an electrocardiogram measured by a device having a different number of leads.

15. A measuring device for recording an electrocardiogram, the device comprising
- at least two electrodes for measuring at least one second biological signal, the second biological signal comprises first data and second data, the first data being values measured over a period of time and the second data being values specifying the manner used to measure the first data;
- a memory storing a trained abstraction model of an artificial neural network and an individualized model of an artificial neural network, the artificial neural networks being trained to create a prediction of third data of the second biological signal, wherein the third data labelling at least one medical or technical state connected with the second biological signal; and
- a processing unit comprising one or more processors configured to
- receive the second biological signal; and
- execute a neural network engine, the artificial neural network engine implementing the artificial neural networks using the trained abstraction model and the individualized model for inferring one or more output based on inputs, the one or more output providing a prediction of the third data.
